(19) Europäisches Patentamt
European Patent Office
Office européen des brevets

(11) **EP 2 020 251 A1**

(12) **EUROPEAN PATENT APPLICATION**

(43) Date of publication:
**04.02.2009 Bulletin 2009/06**

(51) Int Cl.:
***A61N 2/00*** (2006.01)

(21) Application number: **07113243.5**

(22) Date of filing: **26.07.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Cuppen, Johannes Josephus Maria
5508 HB Veldhoven (NL)**

(72) Inventor: **Cuppen, Johannes Josephus Maria
5508 HB Veldhoven (NL)**

(74) Representative: **van Looijengoed, Ferry Antoin
Theodorus et al
De Vries & Metman
Overschiestraat 180
1062 XK Amsterdam (NL)**

(54) **Coil structure for electromagnetic stimulation of a process within a living organism, device using such coil structure and method of driving**

(57) A distributed electromagnetic coil structure adapted for applying a time varying electromagnetic field to at least part of a body of a living organism comprising at least a substantially planar first coil sub-structure. The first coil sub-structure comprises at least a first wire of a predetermined shape and a least a second wire of a predetermined shape, wherein said first and second wire are shaped to define a plurality of first cells. The electromagnetic field is generated substantially by the first cells when a current runs through the first coil sub-structure.

Fig 1a

**Description**

Field of the invention

The invention relates to an electromagnetic transducer for stimulating a process within living organisms using electromagnetic fields. In particular, the invention relates to a distributed electromagnetic coil structure for effective stimulation of processes, in particular the immune response, in living organisms in large-area and large-scale applications.

Background of the invention

It is well known that time varying low-energy electric or magnetic fields produce a range of responses in biological systems. Based on these responses various therapeutic or bio-stimulation treatments using low frequency and low-energy electromagnetic fields have been proposed. For example, US 3,890,953 describes a method for the stimulation of bone growth and other tissues. In US 5,183,456 a method for regulation of the growth of cancer cells is described. In US 5,290,409 a method is described in which the transport of several types of ions can be influenced.

An interesting group of studies have demonstrated that human and murine macrophages can be stimulated to higher activity through low frequency electromagnetic field exposure (see Simkó et al., Eur. J. Cell Biol. Vol. 80, 2001, p. 562-566 and Lupke et al., Free Radic. Res. Vol. 38, 2004, p. 985-993). Several authors have demonstrated that the observed production of cytokines, increased immune parameters and stress effects were initiated by exposure to electromagnetic fields. From these studies it was concluded that low field electromagnetic field exposure causes stress at the cellular level, leading to production of cytokines and consequently biological response, including immune response (see Blank et al., Bio-electrochem. and Bioenerg., Vol. 33, p. 109-114, 1992 and Mol. Biol. Cell Vol. 6, p. 466a, 1995; Goodman et al., Bioelectro-chem. and Bioenerg., Vol. 33 p. 115; Simkó et al., J. Cell. Biochem. Vol. 93, 2004, p. 83-92; Monselise et al., Biochem. & Biophys. Res. Com. Vol. 302(2), p. 427-434, 2004; De Bruyn et al., Environ. Res., Vol. 65 (1) p. 149-160, 1994; Markov et al. in Bioelectromagnetics edited by S.N. Ayrapetryan and M.S. Markov (eds.), Springer 2006, p. 213-225).

Proper stimulation of the immune response leads to improved resistance against infectious diseases and thus positively affects the health of the exposed organism. This insight opens new possibilities for (preventive) treatment of large, dense populations wherein infectious diseases are an increasing problem. Such problems are especially prevalent in populations with genetically uniform organisms such as farmed livestock, chicken, shrimp and fish populations. Infectious diseases can be very damaging to such populations and treatments are very costly.

WO 03/035176 describes a device which is particularly effective in stimulation of the immune system of humans and animals. This device is adapted to the application of time dependent electromagnetic fields to a part of the body of a living organism using a pair of Helmholtz coils. The applied signal has a spectrum of frequencies in which some frequencies or frequency areas are more strongly present than others. Such a device can support a therapy in which afflictions involving inflammation and infection can be treated.

The system for electromagnetic stimulation described in WO 03/035176 is a small-scale system only suitable for the treatment of a single organism. Effective electromagnetic stimulation of large populations on a large-scale and the problems related thereto are not addressed in the prior art. For instance, livestock populations are usually kept in large-area buildings or other spaces of large dimensions. Usually stables and sheds for cows, chickens and pigs or ponds for breeding fish have typical dimensions of at least tens to hundreds of meters. Without special measures, controlled electromagnetic stimulation of such large areas is difficult and would require large amounts of energy. Moreover, when using the device in more remote areas a battery fed system with a solar and/or wind energy supply is necessary. In that case reduction in power consumption is a very important aspect.

Moreover, the buildings where livestock is kept vary in size and construction. The transducers installed in such buildings are tailor made. Consequently, the impedance of these electromagnetic transducers will - to a certain extent - vary from building to building. Consequently, these variances and deviations in the load of the driving electronics of the electromagnetic transducers will affect the electromagnetic signal produced. This will negatively influence the effectiveness of the stimulation treatment which is very dependent on the shape of the signal.

Object of the invention

It is an object of the invention to reduce or eliminate at least one of the drawbacks of the prior.art and to provide distributed coil structures adapted for producing a electromagnetic field signal which is suitable for effective stimulation of processes, in particular the immune response, in living organisms in large-area and large scale-applications.

Summary of the invention

The invention relates to the observation that the area in which the living organisms are kept and to which the electromagnetic field should be applied is in principle spatially limited to a volume of certain dimensions wherein the horizontal (area) dimensions are much larger than the vertical (height) dimensions. The distributed electromagnetic coil structure of the present invention effectively "divides" this volume in a set of sub-volumes of a predetermined size and allows to expose at least a subset of these sub-volumes to the electromagnetic field at different points in time. Division over sub-volumes and exposure of sets of mutually non-adjacent sub-volumes to a time varying electromagnetic field at different points in time efficiently reduces and spreads the total energy requirement for complete exposure of all animals present in the volume over a certain time period. It thus allows the use of simple and cheap electronics for driving the transducers. In this way the electromagnetic field can be applied to the living organisms present in this volume (such as livestock, chickens, fish or shrimps) in an (energy) efficient manner.

One aspect of the invention relates to a distributed electromagnetic coil structure coil comprising at least a substantially planar first coil sub-structure. This coil sub-structure comprises at least a first wire and at least a second wire of a predetermined shape. The first and second shaped wires are arranged with respect to each other to define a plurality of first cells. The electromagnetic field is generated by said first cells if a current runs through said first coil sub-structure.

By defining such cells the electromagnetic field is localized and effectively applied to small sub-volumes of the total volume. The division of the volume is thus effectuated by the particular shape and arrangement of the cells of the distributed coil structure. It is appreciated that in the context of the present invention regarding coil structures distributed over a large area, the term planar means that the dimensions of the coil structure in two dimensions are substantially larger than the third dimension.

In a further aspect of the invention the coil structure further comprises at least one substantially planar second coil sub-structure defined by said second wire of said first coil sub-structure and at least a third wire of a predetermined shape, wherein said second and third wire are shaped to define a plurality of second cells. The electromagnetic field is generated substantially within these second cells if a current runs through said second coil sub-structure. The second coil sub-structure forms a structure which is complementary to the first coil sub-structure. The use of the first and second coil sub-structures allows each sub-volume of the total volume in which the living organisms are kept to be exposed to the electromagnetic field by applying current to the first and second coil sub-structures at different points in time. Such a distributed coil structure thus provides an advantageous and energy efficient way of applying an electromagnetic field to a large area.

Another embodiment of the invention relates to a distributed electromagnetic transducer for applying a time varying electromagnetic field to large population of living organisms. These organisms are kept in a large area S. Typical dimensions of this area correspond to the dimensions of stables for livestock or (breeding) ponds for fish. The distributed transducer comprises at least a first coil structure and a second coil structure, which are - depending on the situation - arranged under or in or above a volume, which is defined by the large area S and a predetermined height of dimensions much smaller than the dimensions defining said large area S. The first coil structure and second coil structure in this embodiment comprise at least a wire with a (spatially) distributed shape.

In one embodiment the (spatially) distributed shape of the wire could be a meandering or a block-wave-shaped or a sawtooth-shaped wire extending under or in or over the volume. Alternatively, the (spatially) distributed shaped wire can be arranged to form a number of loops to form cells. The magnetic flux generated in these loops will be proportional to the current through the wire. Increasing the number of windings in one loop will increase the magnetic field generated by that loop in a simple way without the need to increase the current through the wire.

The coils can be driven by suitable driving electronics, which produce a time varying drive signal. In use, the distributed coil structure is driven such that in a first time period the first coil exposes a first set of sub-volumes of the total volume to a time varying electromagnetic field and in a second period - different from said first time period - the second coil exposes for a second time period a second set of sub-volumes of the total volume to a time varying electromagnetic field. The first set and second set of sub-volumes cover different sub-volumes in the (total) volume. It is appreciated that one or more sub-volumes of the first and second set may partly overlap. The arrangement of such distributed coils allow simple and effective exposure of large populations of animals which are kept in a volume with a large area.

In another embodiment a first, second and third wire are of a predetermined shape and forms an electromagnetic coil structure which is adapted for applying a time varying electromagnetic field to living organisms over a large area. In such coil structure the first, second and third wire is shaped to define a plurality of first cells and second cells, wherein the electromagnetic field is generated substantially by the first cells when current runs through the first and second wire and the electromagnetic field is generated substantially by the second cells when a current runs through the second and third wire.

The shape of the cells can be adapted by adapting the shape of the wires which define the cells. The shape of one or more of the wires can be e.g. substantially meandering or block-wave-shaped or sawtooth-shaped to define substantially circular-, square- or rectangular shaped, diamond-shaped or triangular shaped cells. One or more wires in the distributed transducer can be shaped to form a number of "loops" wherein each loop has one or more windings. Moreover, distributed shapes of the wires of the coil structures of the present invention are not limited to planar structures but also include three dimensional coil structures. For example, it is appreciated that the shape of the wires - and thus the shape of the cells - can be adapted to the shape of the support structure of the wires. Such support structure can comprise an existing support frame in the ceiling, floor or subdivisions in a stable, such as the boxes in which livestock is kept or held during feeding.

The area of the cells can be chosen from a range between 0,01 to 100 m$^2$, in particular between 0,1 and 20 m$^2$ so that large areas can be effectively treated.

In one embodiment of the invention the distributed coil structure of the present invention extends over a surface area wherein the plane of said first coil sub-structure is substantially parallel to said surface area. By arranging a plurality of first coil sub-structures in the plane of said first coil sub-structure, the distributed electromagnetic coil structure is positioned over or under the volume which contains the living organisms to be treated. The advantage of such arrangement is that the coil structure allows free movement of the animals and workers within the volume to be subjected to the electromagnetic field.

In another embodiment of the invention the distributed coil structure extends over a surface area wherein the plane of the first coil sub-structure is substantially perpendicular to said surface area. By arranging a plurality of first sub-coil structures or a plurality of first and second sub-coil structures side by side or next to each other, the distributed electromagnetic coil structure is positioned within the volume which contains the living organisms to be treated. This coil structure allows to make use of the existing frames of boxes for the animals as support frames for the distributed electromagnetic coils.

Another aspect of the invention relates to a system adapted to produce electromagnetic stimulation of processes within living organisms when applied to at least a part of a body of said organisms, which comprises at least one distributed electromagnetic coil structure.

The wires used in the above described embodiments of the invention can be multi-core wires, wherein each wire can be driven by a separate driving amplifier. This way the magnetic field generated by the distributed electromagnetic coil structure can be easily increased without the need to increase the current through the wires.

A further aspect of the invention relates to a system for applying an electromagnetic field to at least part of a body of a living organism. The system comprises at least a distributed electromagnetic coil structure for applying a time varying electromagnetic field to large population of living organisms kept in or spread over a large area S, a generator for applying a time varying signal to the distributed electromagnetic coil structure and attracting means for attracting and/or restricting living animals to the vicinity of the coil structure.

Such attracting means for attracting living animals can be used in systems wherein the living organisms are free to move over a large area and can comprise a fresh water or food supply or a light source or a milking robot or a combination of these features. The attracting means keeps the animals within the vicinity of the electromagnetic coil structure so that these animals can be efficiently exposed to the electromagnetic field. The use of the attracting means thus allows efficient large scale electromagnetic stimulation without the need that the transducer covers the complete area where the living organisms are kept.

Another aspect of the invention relates to a device for applying an electromagnetic field to at least part of a body comprising at least a transducer and a generator for applying a time varying signal to the transducer. The transducer comprises at least one distributed electromagnetic coil structure of the present invention.

Yet another aspect of the invention relates to a method driving at least one distributed electromagnetic coil structure of the present invention. By applying at different periods in time currents to the sub-structures in the electromagnetic coil structure the large area volume containing the living organisms can be effectively subjected to a time-varying electromagnetic field.

It is appreciated that the invention is not limited to the above described embodiments. For instance, the invention also relates to structures, for instance a building, for keeping living organisms comprising at least one distributed electromagnetic coil structure according to the present invention.

Moreover, the invention also relates to a device for applying an electromagnetic field adapted to produce electromagnetic stimulation of processes within a living organism when coupled to at least part of a body of said organism, comprising:

- driving means for generating a time varying drive signal,
- at least one transducer responsive to said drive signal for generating a time varying signal B(t) comprising said electromagnetic field, and

wherein said signal B(t) contains a superposition of one periodic signal $b_i(t)$ or a superposition of at least two periodic

signals $b_i(t)$ (i=1,2,3,...), each signal $b_i(t)$ being defined as:

$$b_i(t) = a_i * (2\exp(-\omega_o t) - (1+\exp(-\omega_o T_i/2)))$$

for $0 \leq t \leq T_i/2$

$$b_i(t) = -b_i(t-T_i/2)$$

for $T_i/2 \leq t \leq T_i$

wherein $T_i$ is the period of $b_i(t)$, $a_i$ is an amplitude of $b_i(t)$ and $\omega_o$ is a characteristic frequency determining the shape of the signal $b_i(t)$, and wherein said transducer further comprises at least one distributed electromagnetic coil structure according to the present invention.

It was experimentally determined that the use of an electromagnetic signal based on the base signals $b_i(t)$ (i=1,2,3,...), which have a special shape, is especially effective in the electromagnetic stimulation of living organisms. The electromagnetic signal B(t) comprises one base signal $b_i(t)$ or, preferably, a superposition of at least two different base signals $b_i(t)$. Devices generating such signals are further described in a related application with title "Device and method for electromagnetic stimulation of a process within living organisms", which is hereby incorporated by reference in this application.

Typically, the amplitude $a_i$ (i=1,2,3, ...) is chosen such that the peak amplitude of B(t) at the treatment positions will be in the range between 1 nT to 1 mT, preferably within the range between 0.01 $\mu$T to 10 $\mu$T. Due to the effective shape of signal B(t) for electromagnetic stimulation, even small amplitudes signals will be sufficient to generate advantageous stimulation. The use of this signal thus drastically reduces energy consumption in large area and large scale applications.

Brief description of the drawings

The invention will be further explained by means of the description of exemplary embodiments, reference being made to the following figures:

Fig. 1 represents schematic drawings of embodiments of the invention.
Fig. 2 represents a schematic drawing of an embodiment of the invention wherein a distributed coil structure is positioned over a surface area and wherein the plane of the coil sub-structures are in parallel with the surface area.
Fig. 3 represents a schematic drawing of an embodiment of the invention wherein a distributed coil structure is positioned over a surface area and wherein the planes of the coil sub-structures are perpendicular to the surface area.
Fig. 4 illustrates exemplary geometrical wire arrangements forming "cells" used in the coil structures of the invention.
Fig. 5 represents a schematic drawing of another embodiment of the invention.
Fig. 6 illustrates a system for electromagnetic stimulating a process in a living organism.
Fig. 7 illustrates an example of distributed coil arrangement for use in stables.
Fig. 8 represents a schematic drawing of the shape of a preferred periodic base signal $b_i(t)$.
Fig. 9 illustrates the results of in vivo experiments on infected fantail goldfish treated with an electromagnetic field signal.

Description of exemplary embodiments

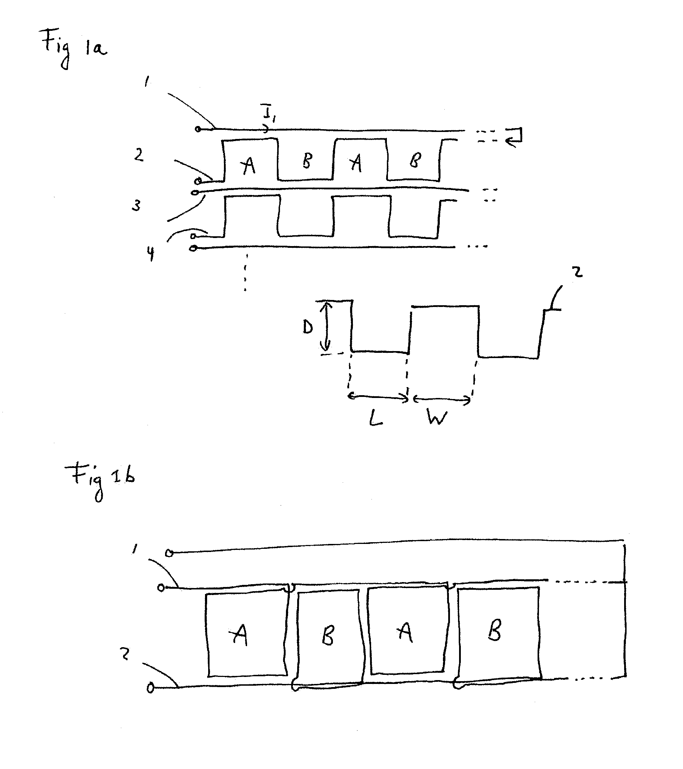

Fig. 1a and 1b schematically show examples of distributed electromagnetic coil configurations according to the present invention comprising a number of wires of a predetermined shape. In the particular embodiment of Fig. 1a the first wire 1 is a substantially straight wire and an the second wire 2 is a meandering wire having a number of blocks characterized by dimensions D,L and W. Wires 1 and 2 are arranged with respect to each other such that parts of wire 2 are arranged over a length W in parallel and in close vicinity to wire 1. Wire 1 and 2 thus form a substantially planar first coil sub-structure comprising a number of substantially closed first areas or cells denoted in Fig. 1a by the letter B. In this embodiment the area of the cell is determined by the dimensions of the meandering pattern and can be varied by changing the dimensions D,L and W of the meandering wire 2. By connecting one end of wires 1 and 2 to each other and the other end of the wires to driving circuitry a current $I_1$ can be applied to the first coil sub-structure as depicted in Fig. 1a. It is appreciated that - when electrically connected to each other - the first coil-substructure (comprising wire 1 and 2) in Fig. 1a can be constructed by the geometrical arrangement of only one wire.

The current will generate a magnetic field around the wires in the coil sub-structure. The main part of the generated field will be localized substantially within and in the vicinity of the cells B. The distributed arrangement of this coil sub-structure thus allows the application of an electromagnetic field over a large number of cells, thus effectively covering a large area.

In a further embodiment a third wire 3 can be arranged to the first and second wire of the first coil-substructure. In the embodiment as illustrated in Fig. 1a, wire 3 is a substantially straight wire arranged next to and parallel with the meandering second wire 2. Wire 2 and 3 thus form - in the same way as wire 1 and 2 - a second coil sub-structure which forms a complement structure of the first coil sub-structure. The second coil sub-structure has a number of cells A which are arranged in between cells B of the first coil-substructure. Wire 2 and 3 can be connected to driving circuitry allowing to apply a current $I_2$ to the second coil sub-structure thereby generating a magnetic field which will be localized substantially within and in the vicinity of the cells A. The distributed coil structure formed by wires 1, 2 and 3 thus forms a coil structure which allows efficient electromagnetic stimulation treatment of the whole area covered by the structure by alternately applying a current to the first coil sub-structure and the second coil sub-structure.

In another embodiment a number of first coil sub-structures can be arranged next to each other effectively forming large area distributed coil structure. This is illustrated in Fig. 1a by wires 1 and 2 and wires 3 and 4, which form two first coil sub-structures. By applying currents to pairs of wires (i.e. wires 1 and 2, wires 2 and 3, wires 3 and 4) an electromagnetic field will be generated which will be localized substantially within and in the vicinity of the cells A, B, C or D. In that way the structure in Fig. 1a can be extended to distributed coil structures comprising a large number of such coil sub-structures.

In Fig. 1b a variant of the coil structure in Fig. 1a is schematically shown. In this embodiment wires 1 and 2 are shaped to form a number of "loops" having one or more windings. As shown in Fig. 1b the loops of wire 1 and wire 2 are arranged to form an interleaved distributed coil structure forming a plurality of loops or cells similar to the coil structure in Fig. 1a. Increasing the number of windings in a loop will increase the magnetic field generated by that loop without increasing the current through the wire. By applying a current to wire 1 and wire 2 at different points in time an electromagnetic field is generated by the loops corresponding to the respective wire. The return paths of wires 1 and 2 can be combined to one wire as shown in Fig. 1b.

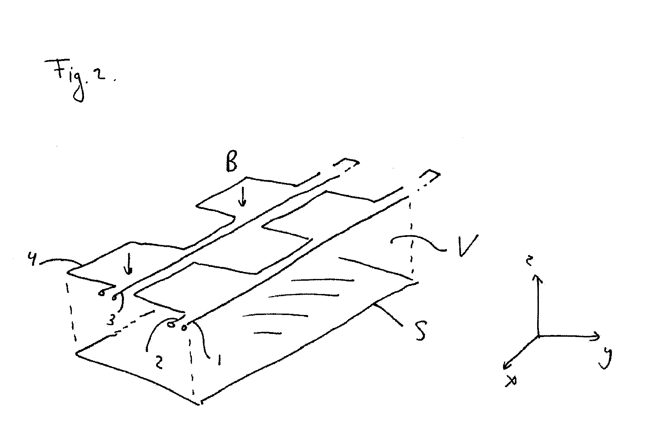

In Fig. 2 the large area distributed coil structure of Fig. 1 is arranged at a certain height above or below a surface area S thus defining a large area volume V wherein the horizontal (area) dimensions are much larger than the vertical (height) dimensions in which the living organisms are kept and to which the electromagnetic field should be applied. The cells of the distributed electromagnetic coil structures of the invention divide the volume in sub-volumes of a predetermined size and allow application of the electromagnetic field to these sub-volumes at different points in time. The distributed coil structure effectively applies electromagnetic field to the living organisms present in this volume, such as livestock, chickens, fish or shrimps in an (energy) efficient manner.

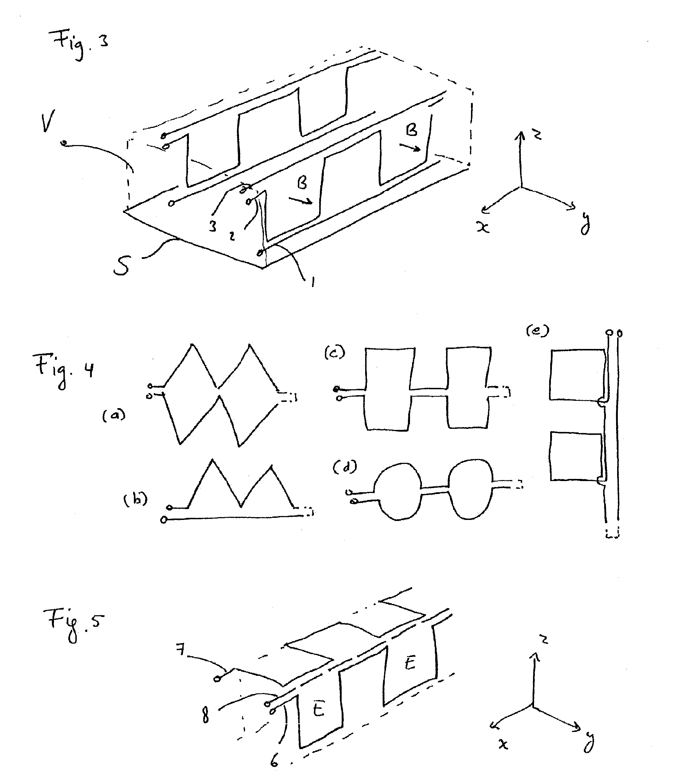

Fig. 3 illustrates another embodiment of an large area distributed coil structure. In this embodiment a number of first coil sub-structures as discussed in connection with Fig. 1 are arranged over a surface area S. The first coil-substructures are arranged side by side with their plane perpendicular to the surface area thus dividing the large area volume V into sub-volumes of a height which is determined by the dimension D of the meandering block-wave shaped wire. Such coil structure allows to make use of the existing frames of boxes in the stables as support frames for the distributed electromagnetic coils.

In the large area coil structures some coil sub-structures can have larger cells than other coil sub-structures. For instance in some applications it is necessary to reduce stray fields generated at the edge of such large area coil structures. The reduction of stray fields is necessary to reduce unwanted and/or uncontrolled exposure to electromagnetic field of objects or living subjects in the vicinity of the large area coil structure. In that case the shape and the orientation of the coil sub-structure at the edges of the large area distributed coil structure can be optimized in order to reduce these stray fields.

In Fig. 1a a substantially planar structure is shown. Here, the term planar should be interpreted in the context of large area distributed coil structures which can cover hundreds of square meters. In such large area structures the wires can deviate from a two dimensional plane, for example by the sagging of the wires under its own weight. Consequently, the term planar structure should be interpreted as a structure having two dimensions which are much larger than the third dimension.

It is appreciated that the geometry of the wire or wires forming the cells can be in various shapes and sizes. In Fig. 4 a-e a non-limiting number of cell geometries, such as diamond shaped, triangular shaped, square shaped and circular shaped cells, is shown. It is also possible to form cells by creating a number of "loops" in a wire as illustrated in Fig. 4e. Increasing the number of windings in a loop will increase the magnetic field generated by the loop. Consequently, by choosing different numbers of windings in each loop, the magnetic field generated by each loop can be varied along the distributed coil structure. Such arrangement allows the application of different field strengths in one treatment session. The different geometries can be used depending on the requirements of a specific situation.

Fig. 5 schematically shows another example of a distributed electromagnetic coil configuration according to the present invention. In this configuration the distributed coil structure comprises two distributed coils wherein each coil is a substantially planar meandering block-wave shaped wire 6. The first and a second substantially planar meandering block-wave shaped wires 6 and 7 are arranged with respect to each other, i.e. shifted with respect to each other over half a period of the block-wave of the wires, to form a number of cells E. In this particular arrangement the planes of wires 6 and 7 are oriented under an angle of substantially 90 degrees. Other orientations however are also possible. When applying a current through wires 6 and 7 in a similar way as illustrated in Fig. 1a a magnetic field will be generated wherein the main part of the generated field will be localized substantially within and in the vicinity of the cells E. This structure differs from the coil structure in Fig. 1a in that the pair of wires 6 and 7 does not form a planar sub-structure. In the embodiment of Fig. 5 an additional straight wire 8 can be arranged on the intersecting line of the planes of the meandering wires 6 and 7 thereby forming a three wire distributed coil structure which can be driving in the same way as the structure as discussed in relation to Fig. 1.

Typically the area of the cells can be chosen from a range between 0,01 to 100 m$^2$, in particular between 0,1 and 20 m$^2$. Such sizes allow large areas to be effectively treated. Moreover, these area sizes correspond to typical dimensions of structures in buildings in which the living organism are kept, such as ceiling support structure or boxes in which livestock is held during feeding or breeding. These structures can be used as support for the distributed coil structures.

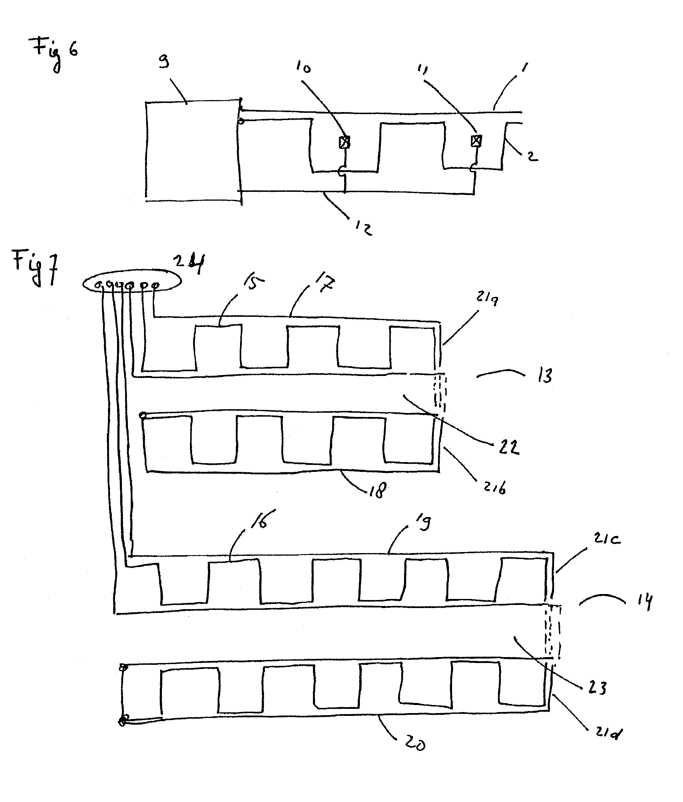

Fig. 6 schematically shows a system for stimulating a process within living organisms using electromagnetic field. The system comprises electronics 9 for generating a driving signal coupled to one or more distributed electromagnetic coil structures of the present invention. If the coil is not a large area distributed coil or if the distributed coil structure does not cover the whole area in which the living organisms are kept, the system can comprise one or more attracting means 10,11 for attracting the animals under or over or at least within the vicinity of the cells. The attracting means can be electronically connected 12 to the driving circuitry of the electromagnetic coils such that feeding and the electromagnetic stimulation can be synchronized.

In case of fish or shrimps in a large area breeding pond such attracting means can be an automated fresh water supply, an automated blower or another automated local oxygenation means, a food supply, a lamp or a combination of such measurers. In case of livestock in a stable the attracting means can be an automated food or drink supply or a milking robot. The use of the attracting means thus allows efficiently large scale electromagnetic stimulation without the need that the coil structure covers the complete area where the living organisms are kept.

In Fig. 7 illustrates a schematic of a distributed coil structure used in a typical experimental set-up. The basic structure of the coil is similar to the one illustrated in Fig. 1 and covers part of the area of two stables 13,14. The meandering wires 15 and 16 and the wires 17,18,19,20 are arranged such that they cover the elongated stable boxes 21a, 21b, 21c, 21d arranged at the opposite sides of the central aisle 22, 23 of each stable. In this particular arrangement, the distributed coil structures of each stable are constructed using only three wires 15,17,18 for the first stable 13 and three wires 16,19,20 for the second stable. Further, the arrangement of the wires is chosen such that the wires can be connected at one central point 24 to suitable driving electronics.

Effective stimulation of processes in living organisms with low field strength fields produced by distributed coil structures is dependent on the shape of the applied electromagnetic field. One aspect of the invention involves the recognition that a signal comprising a superposition of one or more, but preferably at least two periodic electromagnetic signals of a particular shape is especially effective in the stimulation of biological processes, including stimulation of the immune system. The electromagnetic signal produced by the system preferably comprises a time-varying electromagnetic signal B(t) which comprises a spectrum of frequencies in which some frequencies or frequency areas are more strongly present than others.

In another embodiment B(t) comprises one periodic base signal $b_i(t)$ or a superposition of at least two periodic base signals $b_i(t)$ (i=1,2,3, ...), each base signal $b_i(t)$ being defined as:

$$b_i(t) = a_i * (2\exp(-\omega_o t) - (1 + \exp(-\omega_o T_i/2)))$$

for $0 \leq t \leq T_i/2$

$$b_i(t) = -b_i(t - T_i/2)$$

for $T_i/2 \leq t \leq T_i$

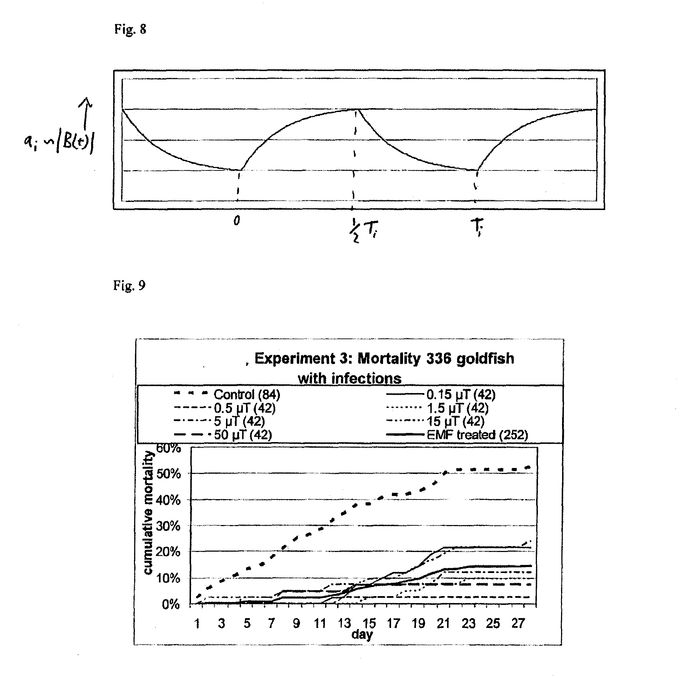

The periodic base signal $b_i(t)$ is depicted in Fig. 8. $T_i$ is the period of the periodic signal $b_i(t)$, $a_i$ is an amplitude

and $\omega_o$ is a characteristic frequency determining the shape of the signal $b_i(t)$. It was experimentally determined that the use of an electromagnetic signal B(t) based on the signals $b_i(t)$ (i=1,2,3,...), which have a special shape, is especially effective in the electromagnetic stimulation of living organisms. Preferably, the characteristic frequency $\omega_o$ substantially matches the R/L ratio of the distributed coil structures, wherein R represents the resistance and L the inductance of the inductive coil(s). Devices generating such signals are further described in a related application with title "Device and method for electromagnetic stimulation of a process within living organisms".

Typically the characteristic frequency $\omega_o$ is chosen from a range between 200 and 20,000 rad.s$^{-1}$, more preferably between 500 and 15,000 rad.s$^{-1}$, in particular between 1000 and 10,000 rad.s$^{-1}$. The amplitude $a_i$ (i=1,2,3,...) is chosen such that the peak amplitude of B(t) at the treatment position will be in the range between 1 nT to 1 mT, preferably within the range between 0.01 $\mu$T to 10 $\mu$T and the periods $T_i$ (i =1,2,3...) are chosen from a range between between 0,01 ms and 1000 ms, preferably between 0,1 ms and 100 ms.

The effectiveness of this signal is shown in a number of in vivo experiments performed on fantail goldfish (Carrassius Auratus spp.). The goldfish were heavily infected with Ecto parasites (Gill parasites) such as Dactylogyrus/Gyrodactylus, Trichodina, Chilodinella and Costia. These types of parasite infections occur frequently at the breeding stage of the fish and increase in frequency during storage and international transport due to the fact that large populations are packaged in one volume. These infections and subsequent secondary bacterial infections cause high mortality if not treated.

Fig. 9. shows typical results from the electromagnetic stimulation treatment for six different field strength ranging from 0.15 $\mu$T to 50 $\mu$T. Every day the fish were exposed for 30 minutes to a time varying electromagnetic field comprising of at least two periodic signals as described in relation to Fig. 7. The results show that the control group suffered a mortality rate up to 52% on day 28. In contrast, the average mortality rate of the treated fish was 15% at day 28. The effectiveness of the treatment reduces slightly when using fields smaller than 0,05 $\mu$T. These results were reproducible and show that the low energy electromagnetic treatment using signals generated by the device of the present invention results in a strong decrease in mortality at all field strength levels used.

The present invention is not limited to large scale electromagnetic stimulation of the immune response in animals, but can also be applied to humans, for instance in the (preventive) treatment against sicknesses such as the bird flue or a biological attack. Moreover, the invention is not limited to the embodiments described above, which may be varied within the scope of the accompanying claims.

**Claims**

1. A distributed electromagnetic coil structure adapted for applying a time varying electromagnetic field to at least part of a body of a living organism, said coil structure comprising at least a substantially planar first coil sub-structure, said first coil sub-structure comprising at least a first wire of a predetermined shape and a least a second wire of a predetermined shape, wherein said first and second wire are shaped to define a plurality of first cells, said electromagnetic field being generated substantially by said first cells when a current runs through said first coil sub-structure.

2. A coil structure according to claim 1, wherein said coil structure further comprises at least one substantially planar second coil sub-structure defined by said second wire of said first coil sub-structure and at least a third wire of a predetermined shape, wherein said second and third wire are shaped to define a plurality of second cells, said electromagnetic field being generated substantially by said second cells when a current runs through said second coil sub-structure.

3. A coil structure according to claim 1 or 2, wherein at least one of said first or second wire comprise a substantially meandering or a block-wave-shaped or a sawtooth-shaped wire.

4. A coil structure according to claims 1 or 2, wherein a plurality of said first coil sub-structures are arranged side by side extending over a surface area and wherein the planes of said coil sub-structures all lie within a plane which is substantially parallel to said surface area.

5. A coil structure according to claims 1 or 2, wherein a plurality of said first coil sub-structures are arranged side by side extending over a surface area and wherein the planes of said coil sub-structures are arranged substantially perpendicular to said surface area.

6. A coil structure according to any of the preceding claims, wherein the area of said cells is chosen from a range between 0,01 to 100 m$^2$, in particular between 0,1 and 20 m$^2$.

7. A distributed electromagnetic transducer for applying a time varying electromagnetic field to large population of living organisms kept in or spread over a large area S, said distributed transducer comprising at least a first coil structure and a second coil structure, said first coil structure and second coil structure are arranged under or in or above a volume defined by said large area S and a predetermined height of dimensions much smaller than the dimensions defining said large area S, wherein said first structure and second coil structure, each comprising at least a wire having a distributed shape, wherein when in use said first coil structure exposes for a first time period a first sub-volume of said volume to a time varying electromagnetic field and said second coil structure exposes for a second time period a second sub-volume of said volume to a time varying electromagnetic field, wherein said first time period and first sub-volume are different from said second time period and second sub-volume respectively.

8. An electromagnetic coil structure adapted for applying over a large area a time varying electromagnetic field to living organisms, said coil structure comprising at least a first, second and third wire of a predetermined spatially distributed shape, said first, second and third wire being shaped to define a plurality of first cells and second cells, wherein when in use said electromagnetic field is generated substantially by said first cells when current runs through said first and second wire and said electromagnetic field is generated substantially by said second cells when a current runs through said second and third wire.

9. System adapted to generate electromagnetic stimulation of processes within living organisms when applied to at least a part of a body of said organisms, comprising at least one distributed electromagnetic coil structure according to any of the preceding claims, said system further comprising attracting means for attracting and/or restricting living animals to the vicinity of at least one of said cells.

10. System for applying a time varying electromagnetic field to a large population of living organisms kept in or spread over a large area, comprising at least a distributed electromagnetic coil structure, and a generator for applying a time varying signal to said coil structure, wherein the system further comprises attracting means for attracting and/or restricting living animals to the vicinity of said coil structure.

11. System according to claim 9 or 10, wherein said attracting means comprises a drink supply or a food supply or a light source or a milking robot or oxygenation means for local oxygen enrichment of water, such as a fresh water supply or an air blower.

12. Device for applying an electromagnetic field to at least part of a body of a living organism comprising at least a transducer and a generator for applying a time varying signal to the transducer, wherein the transducer is adapted for applying the electromagnetic field to at least part of the body and wherein the amplitude of said field is time dependent and comprises a spectrum of frequencies in which some frequencies or frequency areas are more strongly present than others, **characterized in that** said transducer comprises at least one distributed electromagnetic coil structure according to any of claims 1-8.

13. Method of driving a distributed electromagnetic coil structure according to claim 2 or 7 or 8, comprising the steps of:

- applying a first current for a predetermined first time period to said first coil sub-structure such that an electromagnetic field is generated in at least one of said first cells,
- applying a second current for a predetermined second time period to said second coil sub-structure such that an electromagnetic field is generated in at least one of said second cells,

wherein said first and second time period start at different times.

Fig 1a

Fig 1b

Fig. 2.

Fig. 3

Fig. 4

(a)
(b)
(c)
(d)
(e)

Fig. 5

Fig 6

Fig 7

Fig. 8

$$a_i \sim |B(t)|$$

$0$        $\tfrac{1}{2}T_i$        $T_i$

Fig. 9

**Experiment 3: Mortality 336 goldfish with infections**

| | |
|---|---|
| ▪ ▪ ▪ Control (84) | —— 0.15 μT (42) |
| ▪ ▪ ▪ 0.5 μT (42) | ········ 1.5 μT (42) |
| ▪·▪·▪ 5 μT (42) | ▪·▪·▪·· 15 μT (42) |
| — ▪ — 50 μT (42) | ▬▬ EMF treated (252) |

**European Patent Office**

## PARTIAL EUROPEAN SEARCH REPORT

which under Rule 63 of the European Patent Convention shall be considered, for the purposes of subsequent proceedings, as the European search report

Application Number

EP 07 11 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 344 384 A (OSTROW ALVIN [US] ET AL) 6 September 1994 (1994-09-06) | 1-9,11, 12 | INV. A61N2/00 |
| Y | * figure 6 * | 10 | |
| X | US 2002/041185 A1 (KARITA MASAKAZU [JP]) 11 April 2002 (2002-04-11) * figure 1 * | 1-9,11, 12 | |
| A | WO 2006/078715 A (BAUGH CARL E [US]) 27 July 2006 (2006-07-27) * abstract * | 1-9,11, 12 | |
| Y | DE 10 2004 058722 A1 (AIRBUS GMBH [DE]) 14 June 2006 (2006-06-14) * the whole document * | 10 | |
| Y | JP 11 178692 A (NAKAYAMASHIKI IND) 6 July 1999 (1999-07-06) * the whole document * | 10 | |
| Y | WO 01/02051 A (KNOX JO RODNEY [US]; SMITH EDWARD W [US]) 11 January 2001 (2001-01-11) * the whole document * | 10 | **TECHNICAL FIELDS SEARCHED (IPC)** A61N |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 7 March 2008 | Rodríguez Cossío, J |

EPO FORM 1503 03.82 (P04E07)

**European Patent Office**

**PARTIAL EUROPEAN SEARCH REPORT**

Application Number

EP 07 11 3243

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | WO 97/04731 A (PELZ SHMUEL [IL]; THINGINA JULIA [IL]) 13 February 1997 (1997-02-13) * the whole document * ----- | 10 | |
| | | | **TECHNICAL FIELDS SEARCHED** (IPC) |

EPO FORM 1503 03.82 (P04C10)

Claim(s) not searched:
        13

Reason for the limitation of the search (non-patentable invention(s)):

Article 52 (4) EPC - Method for treatment of the human or animal body by therapy

European Patent
Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**European Patent Office**

**LACK OF UNITY OF INVENTION
SHEET B**

Application Number

EP 07 11 3243

The Search Division considers that the present European patent application does not comply with the requirements of unity of invention and relates to several inventions or groups of inventions, namely:

1. claims: 1-9,11,12

    Electgromagnetic coil structure having a substructure comprising first and second wires shaped to define a plurality of cells generating the electromagnetic field.
    ---

2. claim: 10

    System for applying an electromagnetic field to a large population of living organisms comprising attracting means for attracting or restricting living animals to the vicinity of the system.
    ---

## ANNEX TO THE EUROPEAN SEARCH REPORT
## ON EUROPEAN PATENT APPLICATION NO.

EP 07 11 3243

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2008

| Patent document cited in search report | | Publication date | Patent family member(s) | | Publication date |
|---|---|---|---|---|---|
| US 5344384 | A | 06-09-1994 | AT | 218903 T | 15-06-2002 |
| | | | AU | 692429 B2 | 11-06-1998 |
| | | | AU | 5745994 A | 04-07-1994 |
| | | | CA | 2151230 A1 | 23-06-1994 |
| | | | DE | 69332033 D1 | 18-07-2002 |
| | | | DE | 69332033 T2 | 13-02-2003 |
| | | | EP | 0711186 A1 | 15-05-1996 |
| | | | IL | 108036 A | 20-06-1999 |
| | | | WO | 9413357 A1 | 23-06-1994 |
| US 2002041185 | A1 | 11-04-2002 | JP | 2002172177 A | 18-06-2002 |
| WO 2006078715 | A | 27-07-2006 | US | 2007179533 A1 | 02-08-2007 |
| DE 102004058722 | A1 | 14-06-2006 | US | 2006145457 A1 | 06-07-2006 |
| JP 11178692 | A | 06-07-1999 | NONE | | |
| WO 0102051 | A | 11-01-2001 | AU | 6077800 A | 22-01-2001 |
| | | | US | 6267720 B1 | 31-07-2001 |
| WO 9704731 | A | 13-02-1997 | IL | 114728 A | 06-12-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3890953 A
- US 5183456 A
- US 5290409 A
- WO 03035176 A

### Non-patent literature cited in the description

- **SIMKÓ et al.** *Eur. J. Cell Biol.,* 2001, vol. 80, 562-566
- **LUPKE et al.** *Free Radic. Res.,* 2004, vol. 38, 985-993
- **BLANK et al.** *Bio-electrochem. and Bioenerg.,* 1992, vol. 33, 109-114
- *Mol. Biol. Cell,* 1995, vol. 6, 466a
- **GOODMAN et al.** *Bioelectro-chem. and Bioenerg.,* vol. 33, 115
- **SIMKÓ et al.** *J. Cell. Biochem.,* 2004, vol. 93, 83-92
- **MONSELISE et al.** *Biochem. & Biophys. Res. Com.,* 2004, vol. 302 (2), 427-434
- **DE BRUYN et al.** *Environ. Res,* 1994, vol. 65 (1), 149-160
- **MARKOV et al.** Bioelectromagnetics. Springer, 2006, 213-225